# EUROPEAN PATENT SPECIFICATION

(11) **EP 4 481 759 B1**
(45) Date of publication and mention of the grant of the patent: **17.06.2026**
(21) Application number: 24196690.2
(22) Date of filing: 11.04.2019
(51) Int. Cl.: G16H 50/20, G16H 40/63, A61B 5/00, G16H 50/30, G16H 50/70, A61B 5/021, A61B 5/145

(54) **CUSTOMIZED HEALTHCARE MANAGEMENT OF A LIVING SUBJECT**
ANGEPASSTE GESUNDHEITSPFLEGEVERWALTUNG EINES LEBEWESENS
GESTION PERSONNALISÉE DE SOINS DE SANTÉ D'UN SUJET VIVANT

(30) Priority: 12.04.2018 US 201862656686 P; 09.04.2019 US 201916379640
(43) Date of publication of application: 25.12.2024
(62) Divisional of application: 19720252.6
(73) Proprietor: Becton, Dickinson And Company, Franklin Lakes, New Jersey 07417 (US)
(72) Inventor: Hipszer, Brian R., IRVINE, CA, 92614 (US); Al Hatib, Feras, IRVINE, CA, 92614 (US); Jian, Zhongping, IRVINE, CA, 92614 (US)
(74) Representative: Eisenführ Speiser

(56) References cited:
- US-A1- 2008 306 353
- US-A1- 2011 276 341
- US-A1- 2015 138 205
- US-A1- 2015 148 617

## Description

### BACKGROUND

For a patient receiving medical care in an inpatient setting, such as in a hospital for example, a variety of physiological parameters of the patient may be measured and are typically compared to "normal" ranges for those parameters as an aid to diagnosis and to guide treatment. Such "normal" ranges are often determined on the basis of population averages, and while sometimes are adjusted based on the age or gender of the patient, are generally not individualized beyond those sub-population based corrections. Significant variance of one or more of a patient's physiological parameters from established normal ranges may result in interventions designed to normalize the patient's physiological profile by bringing the outlier parameters into the normal range.

However, in certain situations, rather than seeking to normalize a patient's physiological profile, it may be advantageous or desirable to maintain patient homeostasis during medical treatment. For example, maintenance of homeostasis during medical treatment may beneficially avoid taxing the patient's compensatory mechanisms, thereby promoting recovery. Thus, while interventions such as lifestyle change and/or drug therapies designed to bring a patient's physiological parameters into the normal range may be desirable components of a long term treatment strategy for improving general health, maintaining patient homeostasis may be a more effective way to promote recovery in the short term.

US 2015/148617 discloses a method and system for providing an early indication or warning of impending patient deterioration while reducing nuisance alarms, via selection of one of a plurality of early warning algorithms to most accurately provide an early indication or warning, of an impending patient deterioration.

### SUMMARY

There are provided systems and methods for managing healthcare of a living subject, substantially as shown in and/or described in connection with at least one of the figures, and as set forth more completely in the claims.

### BRIEF DESCRIPTION OF THE DRAWINGS

Figure 1 shows a diagram of an exemplary system for managing healthcare of a living subject, according to one implementation;
Figure 2A shows an exemplary implementation for non-invasively sensing one or more physiological parameters at an extremity of a living subject;
Figure 2B shows an exemplary implementation for performing minimally invasive sensing of one or more physiological parameters of a living subject;
Figure 3 shows an exemplary trace of an arterial pressure waveform of a living subject;
Figure 4 shows an exemplary physiological parameter selection pane of a user interface of a system for managing healthcare of a living subject, provided on a display of the system, according to one implementation; and
Figure 5 is a flowchart presenting an exemplary method for use by a system for managing healthcare of a living subject.

### DETAILED DESCRIPTION

The following description contains specific information pertaining to implementations in the present disclosure. One skilled in the art will recognize that the present disclosure may be implemented in a manner different from that specifically discussed herein. The drawings in the present application and their accompanying detailed description are directed to merely exemplary implementations. Unless noted otherwise, like or corresponding elements among the figures may be indicated by like or corresponding reference numerals. Moreover, the drawings and illustrations in the present application are generally not to scale, and are not intended to correspond to actual relative dimensions.

The present application discloses systems and methods for healthcare management of a patient or other living subject (hereinafter "living subject") that overcomes the deficiencies in the conventional art. The present healthcare management solution does so by customizing treatment based on baseline values of one or more physiological parameters of the living subject even when those values deviate from population based norms.

By determining custom target ranges for one or more physiological parameters based on medical history data of the living subject, and storing the custom target ranges in a medical profile of the living subject, the present application discloses a healthcare management solution that customizes treatment goals to maintain homeostasis in the living subject during recovery. In an exemplary implementation, each patient has a medical profile that stores custom target range or ranges 114 unique to each patient and determined based on one or more physiological parameters of each particular patient. In addition, by obtaining present measurements of the one or more physiological parameters, comparing the present measurements to the custom target ranges, and rendering the comparison on a display, the present healthcare management solution advantageously enables the detection of undesirable deviations from homeostasis. As a result, the present application discloses a customized healthcare management solution that advantageously avoids taxing the compensatory mechanisms of the living subject that would be activated if homeostasis were not maintained.

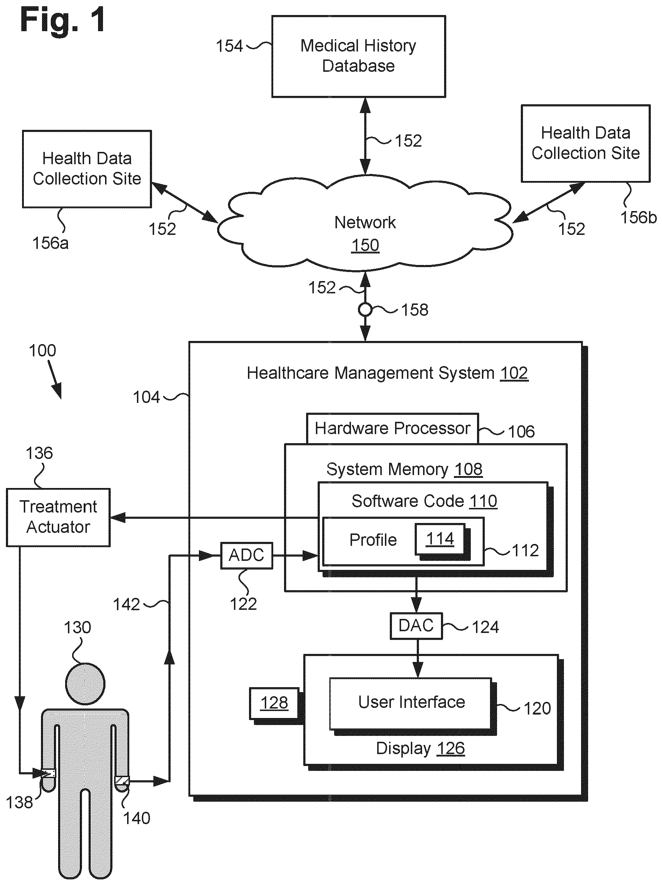

Figure 1 shows a diagram of an exemplary system for managing healthcare of a living subject, according to one implementation. Healthcare management system 102 is implemented within inpatient care environment 100, which may be an intensive care unit (ICU) or hospital treatment venue, for example. As shown in Figure 1, in addition to healthcare management system 102, inpatient care environment 100 includes living subject 130 receiving healthcare management, treatment actuator 136, treatment delivery device 138, physiological sensing device 140, and sensing signals 142.

In addition, Figure 1 shows first and second health data collection sites 156a and 156b, medical history database 154, and medical history data 158. Also shown is communication network 150 communicatively coupling healthcare management system 102 with one or more of medical history database 154 and first and second health data collection sites 156a and 156b via network communication links 152.

As further shown in Figure 1, healthcare management system 102 includes computing platform 104 having hardware processor 106 and system memory 108 implemented as a non-transitory storage device. According to the present exemplary implementation, system memory 108 stores software code 110 including medical profile 112 of living subject 130, which in turn stores custom target range or ranges 114 for one or more physiological parameters of living subject 130. Healthcare management system 102 also includes sensory alarm 128, and display 126 providing user interface 120. In addition, healthcare management system 102 includes analog-to-digital converter 122 (hereinafter "ADC 122") and digital-to-analog converter 124 (hereinafter "DAC 124") used by healthcare management system 102 to process data associated with the provision of customized healthcare management to living subject 130.

It is noted that, although the present application refers to software code 110 as being stored in system memory 108 for conceptual clarity, more generally, system memory 108 may take the form of any computer-readable non-transitory storage medium. The expression "computer-readable non-transitory storage medium," as used in the present application, refers to any medium, excluding a carrier wave or other transitory signal that provides instructions to hardware processor 106 of computing platform 104. Thus, a computer-readable non-transitory medium may correspond to various types of media, such as volatile media and non-volatile media, for example. Volatile media may include dynamic memory, such as dynamic random access memory (dynamic RAM), while non-volatile memory may include optical, magnetic, or electrostatic storage devices. Common forms of computer-readable non-transitory media include, for example, optical discs, RAM, programmable read-only memory (PROM), erasable PROM (EPROM), and FLASH memory.

Display 126 may take the form of a liquid crystal display (LCD), a light-emitting diode (LED) display, an organic light-emitting diode (OLED) display, or another suitable display screen that performs a physical transformation of signals to light. In various implementations, sensory alarm 128 may be implemented as one or more of a visual alarm, an audible alarm, and a haptic alarm. For example, when implemented to provide a visual alarm, sensory alarm 128 may be invoked as flashing and/or colored graphics shown on display 126. When implemented to provide an audible alarm, sensory alarm 128 may be invoked as any suitable warning sound, such as a siren or repeated tone. Moreover, when implemented to provide a haptic alarm, sensory alarm 128 may cause a hardware element coupled to computing platform 104 to vibrate.

Treatment actuator 136 is implemented as a hardware actuator including one or more pumps and/or one or more valves for controlling the administration of a medicine, medicines, or one or more other therapeutic fluids such as saline or another electrolyte solution to living subject 130. Treatment delivery device 138 may include one or more intravenous (IV) lines or ports, for example, enabling IV delivery of medicines and/or other fluids to living subject 130 by treatment actuator 136.

Physiological sensing device 140 may be a hemodynamic sensor, for example, implemented as a non-invasive or minimally invasive sensor attached to living subject 130, and configured to monitor at least one physiological parameter of living subject 130. As specific examples, physiological sensing device 140 may be configured to monitor one or more of a blood glucose level and a blood pressure level, such as a mean arterial pressure (MAP), of living subject 130.

In one implementation, as represented in Figure 1, physiological sensing device 140 may be attached non-invasively at an extremity of living subject 130, such as at a wrist or finger of living subject 130. Although not explicitly shown in Figure 1, in other implementations, physiological sensing device 140 may be attached non-invasively at an ankle or toe of living subject 130. Figure 1 also shows sensing signals 142 received by healthcare management system 102 from physiological sensing device 140. In one implementation, for example, sensing signals 142 may include an arterial pressure waveform of living subject 130. Healthcare management system 102 and physiological sensing device 140 may be configured such that sensing signals 142 may be received by healthcare management system 102 wirelessly, or via a wired connection with physiological sensing device 140.

In one exemplary implementation, healthcare management system 102 for managing healthcare of living subjects or patients are provided where the system includes system memory 108 storing plurality of medical profiles 112 each for a corresponding one of plurality of living subjects 130, each of the plurality of medical profiles 112 having custom target range 114 for a physiological parameter of the corresponding one of the plurality of living subjects 130, wherein the custom target range is uniquely determined based on the medical history data of the corresponding one of the plurality of living subjects 130. The healthcare management system 102 also includes hardware processor 106 configured to execute a software that performs a method of receiving sensing signals 142 for a living subject from a device configured to monitor a physiological parameter of the living subject, obtaining from the sensing signals 142 a present measurement of the physiological parameter, retrieving the custom target range for the physiological parameter from a medical profile of the plurality of medical profiles corresponding to the living subject, comparing the present measurement of the physiological parameter with the custom target range for the physiological parameter retrieved from the medical profile of the living subject, and providing a result of the comparison of the present measurement of the physiological parameter with the custom target range for diagnosis.

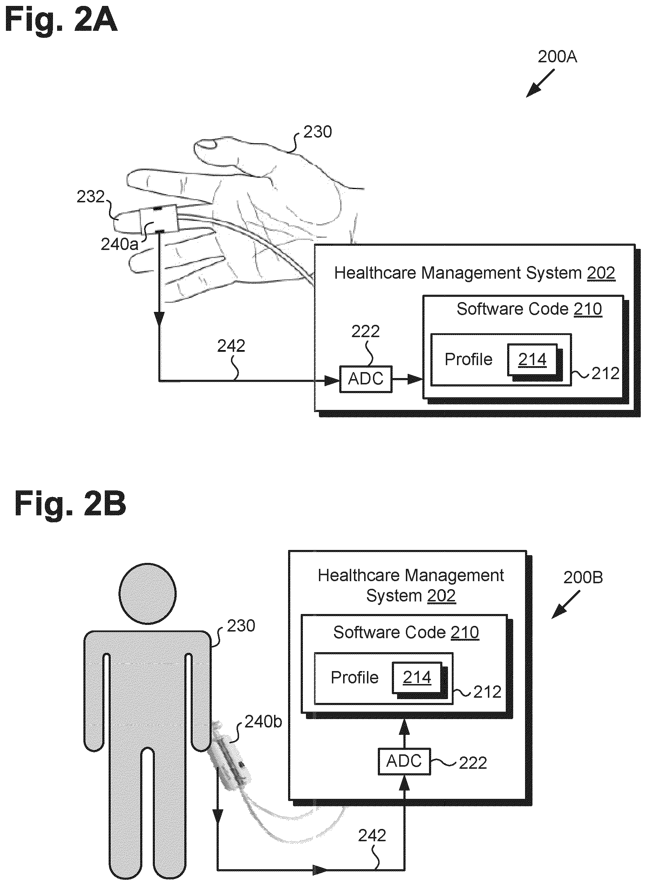

Figure 2A shows an exemplary implementation for non-invasively sensing one or more physiological parameters at an extremity of a living subject. Inpatient care environment 200A, in Figure 2A, shows healthcare management system 202 including software code 210 with medical profile 212 of living subject 230 storing custom target range(s) 214 for one or more physiological parameters of living subject 230. As further shown by Figure 2A, the one or more physiological parameters of living subject 230 is/are detected non-invasively at finger 232 of living subject 230 using physiological sensing device 240a in the form of a sensing cuff. Also shown in Figure 2A are ADC 222 of healthcare management system 202, and sensing signals 242 received by healthcare management system 202 from physiological sensing device 240a.

Living subject 230, sensing signals 242, and physiological sensing device 240a correspond respectively in general to living subject 130, sensing signals 142, and physiological sensing device 140, in Figure 1, and may share any of the characteristics attributed to those corresponding features by the present disclosure. Moreover, healthcare management system 202 including ADC 222 and software code 210 with medical profile 212 storing custom target range(s) 214, in Figure 2A, corresponds in general to healthcare management system 102 including ADC 122 and software code 110 with medical profile 112 storing custom target range(s) 114, in Figure 1, and may share any of the characteristics attributed to that corresponding feature by the present disclosure.

According to the implementation shown in Figure 2A, physiological sensing device 140/240a is designed to sense one or more physiological parameters of living subject 130/230 non-invasively at finger 232 of living subject 130/230. Moreover, as shown in Figure 2A, physiological sensing device 140/240a may take the form of a small, lightweight, and comfortable finger cuff suitable for extended wear by living subject 130/230. It is noted that although physiological sensing device 140/240a is shown as a finger cuff, in Figure 2A, in other implementations, physiological sensing device 140/240a may be suitably adapted as a wrist, ankle, or toe cuff for attachment to living subject 130/230.

It is further noted that the advantageous extended wear capability described above for physiological sensing device 140/240a when implemented as a finger cuff may also be attributed to wrist, ankle, and toe cuff implementations. As a result, physiological sensing device 140/240a may be configured to provide substantially continuous monitoring of living subject 230 over an extended period of time, such as minutes, hours, or days, for example.

Figure 2B shows an exemplary implementation for performing minimally invasive sensing of one or more physiological parameters of living subject 130/230. Inpatient care environment 200B, in Figure 2B, shows healthcare management system 102/202 including software code 110/210 with custom target range(s) 114/214 for one or more physiological parameters of living subject 230 stored in medical profile 112/212. As further shown by Figure 2B, the one or more physiological parameters of living subject 130/230 is/are detected via minimally invasive physiological sensing device 240b. Also shown in Figure 2B are ADC 222 of healthcare management system 202 and sensing signals 242 received by healthcare management system 202 from physiological sensing device 240b.

It is noted that the features shown in Figure 2B and identified by reference numbers identical to those shown in Figure 2A correspond respectively to those previously described features, and may share any of the characteristics attributed to them above. It is further noted that physiological sensing device 240b corresponds in general to physiological sensing device 140, in Figure 1, and may share any of the characteristics attributed to that corresponding feature by the present disclosure.

According to the implementation shown in Figure 2B, physiological sensing device 140/240b is designed to sense one or more physiological parameters of living subject 130/230 in a minimally invasive manner. For example, physiological sensing device 140/240b may be attached to living subject 130/230 via a radial arterial catheter inserted into an arm of living subject 130/230. Alternatively, and although not explicitly represented in Figure 2B, in another implementation, physiological sensing device 140/240b may be attached to living subject 130/230 via a femoral arterial catheter inserted into a leg of living subject 130/230. Like non-invasive physiological sensing device 240a, in Figure 2A, minimally invasive physiological sensing device 240b, in Figure 2B, may be configured to provide substantially continuous monitoring of living subject 230 over an extended period of time, such as minutes, hours, or days, for example.

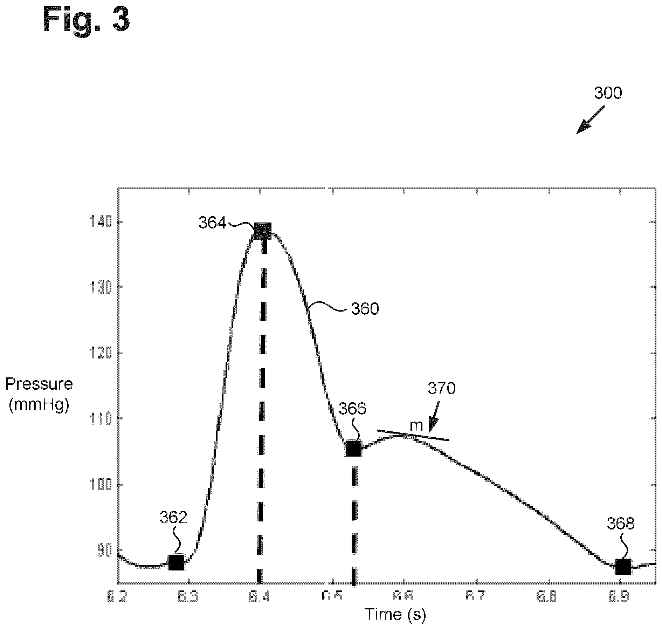

Figure 3 shows an exemplary trace of an arterial pressure waveform of a living subject. Referring to diagram 300, in Figure 3, with additional reference to Figures 1, 2A, and 2B, sensing signals 142/242 may include various indicia included in arterial pressure waveform 360, which may be a central arterial pressure waveform of living subject 130/230, for example. Diagram 300 shows exemplary indicia 362, 364, 366, and 368, corresponding respectively to the start of a heartbeat, the maximum systolic blood pressure level marking the end of systolic rise, the presence of the dicrotic notch marking the end of systolic decay, and the diastole of the heartbeat of living subject 130/230. Also shown by diagram 300 is exemplary slope 370 of arterial pressure waveform 360. It is noted that slope 370 is merely representative of multiple slopes that may be measured at multiple locations along arterial pressure waveform 360.

In addition to indicia 362, 364, 366, and 368 of arterial pressure waveform 360 per se, the behavior of arterial pressure waveform 360 during the intervals between those indicia may also be relevant to the healthcare management of living subject 130/230. For example, the interval between the start of the heartbeat at indicia 362 and the maximum systolic blood pressure level at indicia 364 marks the duration of the systolic rise (hereinafter "systolic rise 362-364"). The systolic decay of arterial pressure waveform 360 is marked by the interval between the maximum systolic blood pressure level at indicia 364 and the dicrotic notch at indicia 366 (hereinafter "systolic decay 364-366"). Together, systolic rise 362-364 and systolic decay 364-366 mark the entire systolic phase (hereinafter "systolic phase 362-366"), while the interval between the dicrotic notch at indicia 366 and the diastole at indicia 368 mark the diastolic phase of arterial pressure waveform 360 (hereinafter "diastolic phase 366-368.")

Also of potential diagnostic interest is the behavior of arterial pressure waveform 360 in the interval from the maximum systolic blood pressure level at indicia 364 to the diastole at indicia 368 (hereinafter "interval 364-368"), as well as the behavior of arterial pressure waveform 360 from the start of the heartbeat at indicia 362 to the diastole at indicia 368 (hereinafter "heartbeat interval 362-368"). The behavior of arterial pressure waveform 360 during intervals: 1) systolic rise 362-364, 2) systolic decay 364-366, 3) systolic phase 362-366, 4) diastolic phase 366-368, 5) interval 364-368, and 6) heartbeat interval 362-368 may be determined by measuring the area under the curve of arterial pressure waveform 360 and the standard deviation of arterial pressure waveform 360 in each of those intervals, for example. The respective areas and standard deviations measured for intervals 1, 2, 3, 4, 5, and 6 above (hereinafter "intervals 1-6") may serve as additional physiological parameters relevant to the healthcare management of living subject 130/230.

As noted above, sensing signals 142/242 may include any or all of indicia 362, 364, 366, 368, and 370, as well as the respective areas and standard deviations measured for intervals 1-6 of arterial pressure waveform 360. In some implementations, the physiological parameters included in sensing signals 142/242 may be utilized to obtain MAP. With respect to MAP, as known in the art, the physiological parameters cardiac output (CO), systemic vascular resistance (SVR), and central venous pressure (CVP) enable the determination of MAP. Specifically, MAP may be obtained by adding CVP to the product of CO and SVR, i.e., MAP = (CO * SVR) + CVP.

Moreover, an approximation of MAP may be advantageously obtained using the physiological parameters diastolic pressure (DP) and systolic pressure (SP), corresponding respectively to the blood pressure level at diastole at indicia 368 of arterial pressure waveform 360, and the maximum systolic blood pressure level at indicia 364. According to such an approximate determination, MAP may be expressed as DP + 1/3(SP - DP).

Thus, in one implementation, sensing signals 142/242 may include at least data corresponding to indicia 364 and 368 for enabling an approximate determination of MAP. However, in other implementations, sensing signals 142/242 may include data for obtaining the physiological parameters CO, SVR, and CVP of living subject 130/230.

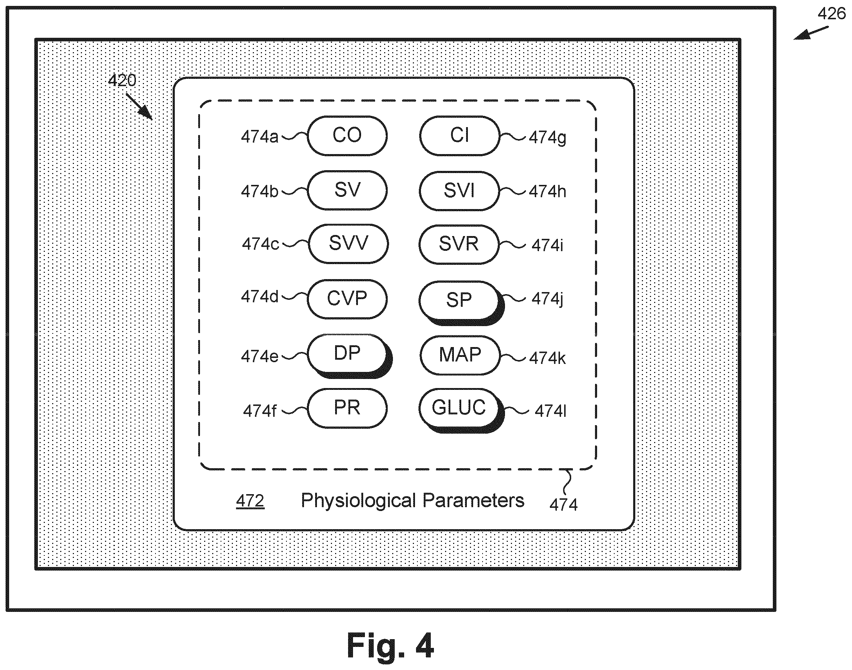

Figure 4 shows an exemplary physiological parameter selection pane presented by a user interface of a system for managing healthcare of a living subject, according to one implementation. Physiological parameter selection pane 472 of user interface 420 is shown on display 426 and enables selection from among a variety of physiological parameters 474. Display 426 and user interface 420 correspond respectively in general to display 126 and user interface 120, in Figure 1, and those corresponding features may share any of the characteristics attributed to either corresponding feature by the present disclosure.

As shown in Figure 4, physiological parameters 474 selectable via user interface 120/420 include CO 474a, stroke volume (SV) 474b, stroke volume variation (SVV) 474c, CVP 474d, DP 474e, pulse rate (PR) 474f, cardiac index (CI) 474g, stroke volume index (SVI) 474h, SVR 474i, SP 474j, MAP 474k, and blood glucose level (GLUC) 474l. It is noted that, in other implementations, physiological parameters 474 may further include additional parameters not represented on physiological parameter selection pane 472.

According to the exemplary implementation shown by Figure 4, user interface 120/420 is implemented as a touch screen user interface. However, in other implementations, user interface 120/420 may be configured to receive inputs for selecting one or more of physiological parameters 474 via a keyboard, via another type of input device, such as a mouse or pressure pad, or as voice commands, for example. As shown by the shadowing of physiological parameters 474e, 474j, and 474l, the blood pressure level parameters DP and SP, and the blood glucose level parameter GLUC have been selected for monitoring using healthcare management system 102/202.

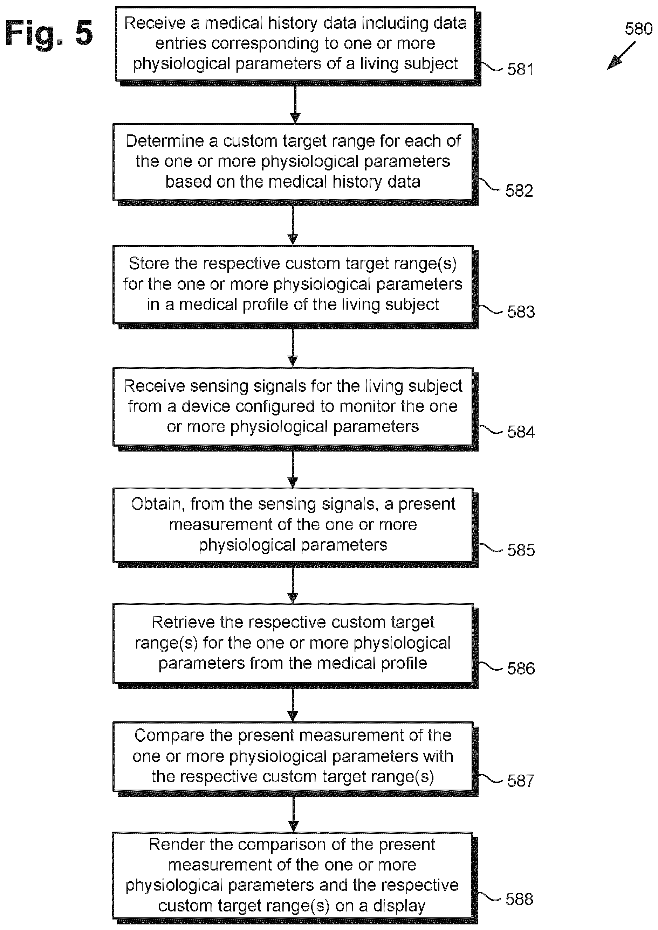

Example implementations of the present inventive concepts will be further described below with reference to Figure 5, which shows flowchart 580 presenting an exemplary method for use by a system for managing healthcare of a living subject. With respect to the method outlined in Figure 5, it is noted that certain details and features have been left out of flowchart 580 in order not to obscure the discussion of the inventive features in the present application. The method outlined in flowchart 580 can be performed by healthcare management system 102/202, using software code 110/210 executed by hardware processor 106 of computing platform 104.

Flowchart 580 begins with receiving medical history data 158 of living subject 130/230, medical history data 158 including multiple data entries corresponding to one or more physiological parameters 474 (action 581). Medical history data 158 may include multiple measurements of one or more of physiological parameters 474, thereby enabling identification of a baseline normal range of values for those physiological parameters relative to homeostasis in living subject 130/230.

In the interests of conceptual clarity, and merely by way of example, the method outlined by flowchart 580 is described below by reference to a specific implementation in which medical history data 158 includes multiple blood pressure level measurements corresponding to physiological parameters DP 474e and SP 474j of living subject 130/230, and multiple blood glucose level measurements corresponding to physiological parameter GLUC 474l, of living subject 130/230. However, it is noted that medical history data 158 may include data entries corresponding to any physiological parameters discussed in the present disclosure, such as MAP 474k or any parameter enabling a determination of MAP, for example. Furthermore, according to the present example implementation, the blood pressure level and blood glucose level measurements included in medical history data 158 are measurements taken prior to hospitalization or other inpatient medical treatment (hereinafter "inpatient treatment") of living subject 130/230.

Where inpatient treatment of living subject 130/230 is preplanned, for example, medical history data 158 may include blood pressure level and blood glucose level measurements taken on several different occasions during the days or weeks preceding inpatient treatment. Alternatively, where inpatient treatment of living subject 130/230 is not preplanned, such as due to accident, injury, or acute illness, for example, medical history data 158 may include blood pressure level and blood glucose measurements taken during prior medical examination or testing of living subject 130/230.

Prior medical examinations of living subject 130/230 may include annual examinations of living subject 130/230 by a primary healthcare provider for living subject 130/230, or may include prior visits by living subject 130/230 to a wellness clinic for routine checking of blood pressure and/or blood glucose levels. Prior medical testing of living subject 130/230 may include testing of living subject 130/230 using automated testing equipment designed to measure and store test results, such as blood pressure levels and/or blood glucose levels. Such automated testing may be performed using publicly accessible testing equipment in a pharmacy, for example, and may also include self-testing by living subject 130/230 at home or elsewhere.

Some of the blood pressure level and/or blood glucose level measurements included in medical history data 158 may be performed at first health data collection site 156a, which may be a doctor's office, a wellness clinic, or a hospital remote from inpatient care environment 100. In addition, or alternatively, some of the blood pressure level and/or blood glucose level measurements included in medical history data 158 may be performed at second data collection site 156b, which may be a pharmacy, school, or private home remote from inpatient care environment 100.

In some implementations, medical history data 158 originating from first and second health data collection sites 156a and 156b may be transmitted to medical history database 154 via communication network 150 and network communication links 152. In those implementations, medical history data 158 may be subsequently transferred from medical history database 154 to healthcare management system 102/202, also via communication network 150 and network communication links 152. In one such implementation, for example, medical history database 154 may correspond to one or more web servers, accessible over communication network 150 in the form of a packet-switched network such as the Internet, for example. Alternatively, communication network 150 may be implemented as a private wide area network (WAN), a local area network (LAN), or another type of limited distribution network.

Alternatively, healthcare management system 102/202 may receive medical history data 158 directly from one or both of first and second health data collection sites 156a and 156b via communication network 150. Medical history data 158 may be received by software code 110/210 of healthcare management system 102/202, executed by hardware processor 106 of computing platform 104.

Flowchart 580 continues with determining custom target ranges 114/214 for each of physiological parameters 474e, 474j, and 474l based on medical history data 158 (action 582). As noted above, medical history data 158 is collected for living subject 130/230 prior to inpatient treatment of living subject 130/230. As a result, medical history data 158 can be analyzed to determine custom target ranges 114/214 for physiological parameters 474e, 474j, and 474l based on blood pressure level and blood glucose level measurements taken when living subject 130/230 is in homeostasis.

In one implementation, for example, an average of DP 474e and SP 474j for living subject 130/230 when in homeostasis can be utilized to determine custom target ranges 114/214 for the blood pressure levels of living subject 130/230 during inpatient treatment. Analogously, an average of GLUC 474l for living subject 130/230 when in homeostasis can be utilized to determine custom target range 114/214 for the blood glucose level of living subject 130/230 during inpatient treatment. For example, medical history data 158 may include results of HbA1c glycated hemoglobin testing of living subject 130/230, enabling determination of a three month average blood glucose level of living subject 130/230. Determination of custom target ranges 114/214 for each of physiological parameters 474e, 474j, and 474l based on medical history data 158 may be performed by software code 110/210 of healthcare management system 102/202, executed by hardware processor 106 of computing platform 104.

Flowchart 580 continues with storing custom target ranges 114/214 for respective physiological parameters 474e, 474j, and 474l in medical profile 112/212 of living subject 130/230 (action 583). As shown in Figure 1, in some implementations, for example when living subject 130/230 is presently receiving inpatient treatment, medical profile 112/212 including custom target ranges 114/214 of physiological parameters 474e, 474j, and 474l may be stored locally in system memory 108.

Alternatively, in implementations in which actions 581, 582, and 583 precede inpatient treatment of living subject 130/230, medical profile 112/212 of living subject 130/230 may be transferred to medical history database 154 for storage via communication network 150 and network communication links 152. Storing of custom target ranges 114/214 for respective physiological parameters 474e, 474j, and 474l in medical profile 112/212 of living subject 130/230 may be performed by software code 110/210 of healthcare management system 102/202, executed by hardware processor 106 of computing platform 104.

Flowchart 580 continues with receiving sensing signals 142/242 for living subject 130/230 from physiological sensing device 140/240a/240b configured to monitor physiological parameters 474e, 474j, and 474l of living subject 130/230 (action 584). Healthcare management system 102/202 may be configured to receive sensing signals 142/242 as analog signals or digital signals. In implementations in which sensing signals 142/242 are received as analog signals, healthcare management system 102/202 utilizes ADC 122/222 to convert sensing signals 142/242 into digital signals. As noted above, sensing signals 142/242 may be received wirelessly, or via a wired connection to physiological sensing device 140/240a/240b. Receiving of sensing signals 142/242 may be performed using software code 110/210 of healthcare management system 102/202, executed by hardware processor 106 of computing platform 104.

Flowchart 580 continues with obtaining, from sensing signals 142/242, a present measurement of physiological parameters 474e, 474j, and 474l (action 585). The present measurement of physiological parameters 474e, 474j, and 474l may be obtained by software code 110/210 of healthcare management system 102/202, executed by hardware processor 106 of computing platform 104.

In some implementations, the physiological parameters DP 474e, SP 474j, and GLUC 474l may be obtained by software code 110/210 directly from sensing signals 142/242. Alternatively with respect to DP 474e and SP 474j, physiological sensing device 140/240a/240b may be used to sense a peripheral arterial blood pressure of living subject 130/230 at an extremity of living subject 130/230. In that implementation, software code 110/210 may obtain DP 474e and SP 474j after transformation of the peripheral arterial blood pressure to a central arterial blood pressure of living subject 130/230.

In implementations in which MAP 474k is monitored by healthcare management system 102/202, MAP 474k may be obtained as an approximation based on DP 474e and SP 474j, as described above. Alternatively and as also described above, MAP 474k may be more precisely obtained based on CO 474a, CVP 474d, and SVR 474i.

Flowchart 580 continues with retrieving custom target ranges 114/214 for respective physiological parameters 474e, 474j, and 474l from medical profile 112/212 of living subject 130/230 (action 586). As noted above, in some implementations, medical profile 112/212 including custom target ranges 114/214 of physiological parameters 474e, 474j, and 474l may be stored locally in system memory 108. In those implementations, retrieval of custom target ranges 114/214 from medical profile 112/212 may be performed as a transfer of data within system memory 108.

Alternatively, in implementations in which medical profile 112/212 is stored on medical history database 154, retrieval of custom target ranges 114/214 for respective physiological parameters 474e, 474j, and 474l may be performed using communication network 150. Retrieval of custom target ranges 114/214 for respective physiological parameters 474e, 474j, and 474l stored in medical profile 112/212 of living subject 130/230 may be performed by software code 110/210 of healthcare management system 102/202, executed by hardware processor 106 of computing platform 104.

Flowchart 580 continues with comparing the present measurements of physiological parameters 474e, 474j, and 474l with their respective custom target ranges 114/214 retrieved from medical profile 112/212 of living subject 130/230 (action 587). Comparison of the present measurements of physiological parameters 474e, 474j, and 474l with their respective custom target ranges 114/214 may be performed by software code 110/210 of healthcare management system 102/202, executed by hardware processor 106 of computing platform 104.

Flowchart 580 can conclude with rendering the comparison of the present measurements of physiological parameters 474e, 474j, and 474l with their respective custom target ranges 114/214 on display 126/426 (action 588). Rendering of the comparison of the present measurements of physiological parameters 474e, 474j, and 474l with their respective custom target ranges 114/214 on display 126/426 may be performed by software code 110/210 of healthcare management system 102/202, executed by hardware processor 106 of computing platform 104. Moreover, in implementations in which rendering of the comparison includes display of an arterial pressure waveform, software code 110/210, executed by hardware processor 106, may utilize DAC 124 to convert digital signals into analog signals for presentation on display 126/426.

Although not included among the actions outlined by flowchart 580, in some implementations, the present method may include invoking, in response to the comparison of action 587, sensory alarm 128 if the present measurement of one or more of physiological parameters 474e, 474j, and 474l is/are outside of respective custom target ranges 114/214. As noted above by reference to Figure 1, sensory alarm 128 may be implemented as one or more of a visual alarm, an audible alarm, and a haptic alarm. For example, when implemented to provide a visual alarm, sensory alarm 128 may be invoked as flashing and/or colored graphics shown by user interface 120/420 on display 126/426. When implemented to provide an audible alarm, sensory alarm 128 may be invoked as any suitable warning sound, such as a siren or repeated tone. When implemented to provide a haptic alarm, sensory alarm 128 may cause a hardware element coupled to computing platform 104 to vibrate.

In addition, in some implementations, the present method may include determining, by software code 110/210 executed by hardware processor 106, a treatment recommendation for living subject 130/230 based on the present measurement of physiological parameters 474e, 474j, and 474l and their respective custom target ranges 114/214. For example, where one or both of DP 474e and SP 474j are higher than their respective custom target ranges 114/214, software code 110/210 executed by hardware processor 106 may determine a treatment recommendation including oral administration of one or more antihypertension medicines to reduce the blood pressure level of living subject 130/230. Examples of such medicines include angiotensin-converting-enzyme (ACE) inhibitors, angiotensin II receptor blockers (ARBs), calcium channel blockers, diuretics, and beta blockers, to name a few.

Alternatively, or in addition, where GLUC 474l is higher than its custom target range 114/214, software code 110/210 executed by hardware processor 106 may determine a treatment recommendation including administration of a medicine such as insulin to lower the blood glucose level of living subject 130/230. Alternatively, one or more orally administered medicines for reducing the blood glucose level of living subject 130/230, such as sulfonylureas, biguanides, meglitinides, and thiazolidinediones, for example may be recommended. Hardware processor 106 may further execute software code 110/210 to render the treatment recommendation on display 126/426.

In some implementations, the present method may further include treating, in response to the comparison of action 587, living subject 130/230 having an increased risk for death or illness within a sufficient lead time to decrease the risk of death or illness for living subject 130/230. For example, hardware processor 106 may execute software code 110/210 to activate one or more hardware actuators of treatment actuator 136 to perform at least a portion of the treatment recommendation for living subject 130/230. As noted above by reference to Figure 1, treatment actuator 136 may include one or more hardware actuators in the form of one or more pumps and/or one or more valves for controlling the administration of a medicine or medicines to living subject 130/230.

As a specific example, where one or both of DP 474e and SP 474j are elevated sufficiently to indicate that living subject 130/230 is suffering a hypertensive crisis, hardware processor 106 may execute software code 110/210 to activate pump(s) and/or valve(s) of treatment actuator 136 to administer antihypertension medicine intravenously via treatment delivery device 138, which may include one or more IV lines or ports. Examples of such intravenously administered antihypertension medicines include sodium nitroprusside, hydralazine, and the beta blocker labetalol.

Thus, the present application discloses systems and methods for customizing healthcare management of a living subject. By determining custom target ranges for one or more physiological parameters based on medical history data of the living subject, and storing the custom target ranges in a medical profile of the living subject, the present application discloses a healthcare management solution that customizes treatment goals to maintain homeostasis in the living subject. In addition, by obtaining present measurements of the one or more physiological parameters, comparing the present measurements to the custom target ranges, and rendering the comparison on a display, the present healthcare management solution advantageously enables the detection of undesirable deviations from homeostasis. As a result, the present customized healthcare management solution avoids taxing the compensatory mechanisms of the living subject that would be otherwise activated if homeostasis were not maintained, thereby advantageously promoting recovery by the living subject.

From the above description it is manifest that various techniques can be used for implementing the concepts described in the present application without departing from the scope of those concepts. Moreover, while the concepts have been described with specific reference to certain implementations, a person of ordinary skill in the art would recognize that changes can be made in form and detail without departing from the scope of those concepts. As such, the described implementations are to be considered in all respects as illustrative and not restrictive. It should also be understood that the present application is not limited to the particular implementations described herein, but many rearrangements, modifications, and substitutions are possible without departing from the scope of the present disclosure as defined by the appended claims

## Claims

1. A hardware processor (106) configured to execute a software code (110) stored in a computer-readable non-transitory storage medium to:
receive medical history data (581) of a living subject, the medical history data including a plurality of data entries corresponding to at least one physiological parameter of the living subject;
determine a custom target range (582) for the at least one physiological parameter based on the medical history data of the living subject, wherein the custom target range (114) is uniquely determined based on medical history data of the corresponding one of the plurality of living subjects taken when the respective living subject is in homeostasis; and
store the custom target range (583) for the at least one physiological parameter in a medical profile (112) of the living subject (130);
receive sensing signals (584) for a living subject from a device configured to monitor a physiological parameter of the living subject;
obtain, from the sensing signals, a present measurement (585) of the physiological parameter;
retrieve the custom target range (586) for the physiological parameter from the medical profile of the living subject;
compare the present measurement (587) of the physiological parameter with the custom target range for the physiological parameter retrieved from the medical profile of the living subject; and
provide a result of the comparison (588) of the present measurement of the physiological parameter with the custom target range for diagnosis.

2. The hardware processor of claim 1, which is further configured to execute the software code to invoke, in response to the comparison, a sensory alarm if the present measurement of the at least one physiological parameter is outside of the custom target range.

3. The hardware processor of any of claims 1 to 2, which is further configured to execute the software code to determine a treatment recommendation for the living subject, in response to the comparison, based on the present measurement of the at least one physiological parameter and the custom target range.

4. The hardware processor of claim 3, which is further configured to execute the software code to render the treatment recommendation on a display (126).

5. The hardware processor of claim 3, which is further configured to execute the software code to activate at least one hardware actuator to perform at least a portion of the treatment recommendation for the living subject.

6. The hardware processor of any of claims 1 to 5, wherein the at least one physiological parameter comprises a blood glucose level of the living subject.

7. The hardware processor of claim 6, which is further configured to execute the software code to administer a medicine to the living subject to reduce the blood glucose level of the living subject when the blood glucose level of the living subject is higher than the custom target range for the blood glucose.

8. The hardware processor of any of claims 1 to 5, wherein the at least one physiological parameter comprises a blood pressure level of the living subject.

9. The hardware processor of claim 8, which is further configured to execute the software code to administer a medicine to the living subject to reduce the blood pressure level of the living subject when the blood pressure level of the living subject is higher than the custom target range for the blood pressure level.

10. The hardware processor of any of claims 1 to 5, wherein the at least one physiological parameter comprises a mean arterial pressure (MAP) of the living subject.

11. The hardware processor of claim 10, wherein the at least one physiological parameter comprises a plurality of parameters enabling determination of the MAP of the living subject.

## Patentansprüche

1. Ein Hardware-Prozessor (106), der ausgebildet ist, einen auf einem computerlesbaren, nichtflüchtigen Speichermedium gespeicherten Softwarecode (110) auszuführen, um:
medizinische Historiendaten (581) eines lebenden Patienten zu empfangen, wobei die medizinischen Historiendaten eine Vielzahl von Dateneinträgen umfassen, die mindestens einem physiologischen Parameter des lebenden Patienten entsprechen;
einen benutzerdefinierten Zielbereich (582) für den mindestens einen physiologischen Parameter auf Grundlage der medizinischen Historiendaten des lebenden Patienten zu bestimmen, wobei der benutzerdefinierte Zielbereich (114) eindeutig auf Grundlage der medizinischen Historiendaten des entsprechenden Patienten aus der Vielzahl von lebenden Patienten bestimmt wird, die erfasst wurden, als sich der jeweilige lebende Patient im homöostatischen Zustand befand; und
den benutzerdefinierten Zielbereich (583) für den mindestens einen physiologischen Parameter in einem medizinischen Profil (112) des lebenden Patienten (130) zu speichern;
Erfassungssignale (584) für einen lebenden Patienten von einem Gerät zu empfangen, das zur Überwachung eines physiologischen Parameters des lebenden Patienten ausgebildet ist;
einen aktuellen Messwert (585) des physiologischen Parameters aus den Erfassungssignalen zu ermitteln;
den benutzerdefinierten Zielbereich (586) für den physiologischen Parameter aus dem medizinischen Profil des lebenden Patienten abzurufen;
den aktuellen Messwert (587) des physiologischen Parameters mit dem aus dem medizinischen Profil des lebenden Patienten abgerufenen benutzerdefinierten Zielbereich für den physiologischen Parameter zu vergleichen; und
ein Ergebnis des Vergleichs (588) des aktuellen Messwerts des physiologischen Parameters mit dem benutzerdefinierten Zielbereich für die Diagnose breitzustellen.

2. Hardware-Prozessor nach Anspruch 1, der weiter ausgebildet ist, den Softwarecode auszuführen, um in Reaktion auf den Vergleich, einen sensorischen Alarm auszulösen, falls der aktuelle Messwert des mindestens einen physiologischen Parameters außerhalb des benutzerdefinierten Zielbereichs ist.

3. Hardware-Prozessor nach einem der Ansprüche 1 bis 2, der weiter ausgebildet ist, den Softwarecode auszuführen, um in Reaktion auf den Vergleich eine Behandlungsempfehlung für den lebenden Patienten zu ermitteln, basierend auf dem aktuellen Messwert des mindestens einen physiologischen Parameters und dem benutzerdefinierten Zielbereich.

4. Hardware-Prozessor nach Anspruch 3, der weiter ausgebildet ist, den Softwarecode auszuführen, um die Behandlungsempfehlung auf einem Display (126) darzustellen.

5. Hardware-Prozessor nach Anspruch 3, der weiter ausgebildet ist, den Softwarecode auszuführen, um mindestens einen Hardware-Aktuator zu aktivieren, um mindestens einen Teil der Behandlungsempfehlung für den lebende Patienten durchzuführen.

6. Hardware-Prozessor nach einem der Ansprüche 1 bis 5, wobei der mindestens eine physiologische Parameter einen Blutzuckerspiegel des lebenden Patienten umfasst.

7. Hardware-Prozessor nach Anspruch 6, der weiter ausgebildet ist, den Softwarecode auszuführen, um dem lebenden Patienten ein Medikament zu verabreichen, um den Blutzuckerspiegel des lebenden Patienten zu senken, wenn der Blutzuckerspiegel des lebenden Patienten höher als der benutzerdefinierte Zielbereich für den Blutzucker ist.

8. Hardware-Prozessor nach einem der Ansprüche 1 bis 5, wobei der mindestens eine physiologische Parameter einen Blutdruckwert des lebenden Patienten umfasst.

9. Hardware-Prozessor nach Anspruch 8, der weiter ausgebildet ist, den Softwarecode auszuführen, um dem lebenden Patienten ein Medikament zu verabreichen, um den Blutdruckwert des lebenden Patienten zu senken, wenn der Blutdruckwert des lebenden Patienten höher als der benutzerdefinierte Zielbereich für den Blutdruckwert ist.

10. Hardware-Prozessor nach einem der Ansprüche 1 bis 5, wobei der mindestens eine physiologische Parameter einen mittleren arteriellen Druck (MAP) des lebenden Patienten umfasst.

11. Hardware-Prozessor nach Anspruch 10, wobei der mindestens eine physiologische Parameter eine Vielzahl von Parametern umfasst, die die Bestimmung des MAP des lebenden Patienten ermöglichen.

## Revendications

1. Processeur matériel (106) configuré pour exécuter un code logiciel (110) stocké dans un support de stockage non transitoire lisible par ordinateur destiné à :
recevoir (581) des données d'antécédents médicaux d'un sujet vivant, les données d'antécédents médicaux comprenant une pluralité d'entrées de données correspondant à au moins un paramètre physiologique du sujet vivant ;
déterminer (582) une plage cible personnalisée pour ledit au moins un paramètre physiologique sur la base des données d'antécédents médicaux du sujet vivant, la plage cible personnalisée (114) étant déterminée uniquement sur la base de données d'antécédents médicaux du sujet vivant correspondant parmi la pluralité de sujets vivants prises lorsque le sujet vivant respectif est en homéostasie ; et
stocker (583) la plage cible personnalisée pour ledit au moins un paramètre physiologique dans un profil médical (112) du sujet vivant (130) ;
recevoir (584) des signaux de détection pour un sujet vivant à partir d'un dispositif configuré pour surveiller un paramètre physiologique du sujet vivant ;
obtenir (585), à partir des signaux de détection, une mesure actuelle du paramètre physiologique ;
récupérer (586) la plage cible personnalisée pour le paramètre physiologique à partir du profil médical du sujet vivant ;
comparer (587) la mesure actuelle du paramètre physiologique avec la plage cible personnalisée pour le paramètre physiologique récupéré à partir du profil médical du sujet vivant ; et
fournir (588) un résultat de la comparaison de la mesure actuelle du paramètre physiologique avec la plage cible personnalisée pour le diagnostic.

2. Processeur matériel selon la revendication 1, qui est en outre configuré pour exécuter le code logiciel afin de provoquer, en réponse à la comparaison, une alarme sensorielle si la mesure actuelle dudit au moins un paramètre physiologique est en dehors de la plage cible personnalisée.

3. Processeur matériel selon l'une quelconque des revendications 1 à 2, qui est en outre configuré pour exécuter le code logiciel afin de déterminer une recommandation de traitement pour le sujet vivant, en réponse à la comparaison, sur la base de la mesure actuelle dudit au moins un paramètre physiologique et de la plage cible personnalisée.

4. Processeur matériel selon la revendication 3, qui est en outre configuré pour exécuter le code logiciel afin d'afficher la recommandation de traitement sur un dispositif d'affichage (126).

5. Processeur matériel selon la revendication 3, qui est en outre configuré pour exécuter le code logiciel afin d'activer au moins un actionneur matériel destiné à effectuer au moins une partie de la recommandation de traitement pour le sujet vivant.

6. Processeur matériel selon l'une quelconque des revendications 1 à 5, ledit au moins un paramètre physiologique comprenant un taux de glycémie du sujet vivant.

7. Processeur matériel selon la revendication 6, qui est en outre configuré pour exécuter le code logiciel afin d'administrer un médicament au sujet vivant destiné à réduire le taux de glycémie du sujet vivant lorsque le taux de glycémie du sujet vivant est supérieur à la plage cible personnalisée pour la glycémie.

8. Processeur matériel selon l'une quelconque des revendications 1 à 5, ledit au moins un paramètre physiologique comprenant un niveau de tension artérielle du sujet vivant.

9. Processeur matériel selon la revendication 8, qui est en outre configuré pour exécuter le code logiciel afin d'administrer un médicament au sujet vivant destiné à réduire le niveau de tension artérielle du sujet vivant lorsque le niveau de tension artérielle du sujet vivant est supérieur à la plage cible personnalisée pour le niveau de tension artérielle.

10. Processeur matériel selon l'une quelconque des revendications 1 à 5, ledit au moins un paramètre physiologique comprenant une tension artérielle moyenne (TAM) du sujet vivant.

11. Processeur matériel selon la revendication 10, ledit au moins un paramètre physiologique comprenant une pluralité de paramètres permettant la détermination de la TAM du sujet vivant.
